# FASCICULE DE BREVET EUROPEEN

(11) **EP 3 324 943 B1**
(45) Date de publication et mention de la délivrance du brevet: **23.06.2021**
(21) Numéro de dépôt: 16750963.7
(22) Date de dépôt: 15.07.2016
(51) Int. Cl.: A61K 9/16, A61K 9/20, B01J 2/00

(54) **GRANULES DE MANNITOL POUR COMPRESSION DIRECTE**
MANNITOLGRANULAT ZUR DIREKTEN VERPRESSUNG
MANNITOL GRANULES FOR DIRECT COMPRESSION

(30) Priorité: 17.07.2015 FR 1556787
(43) Date de publication de la demande: 30.05.2018
(73) Titulaire: Roquette Frères, 62136 Lestrem (FR)
(72) Inventeur: BOIT, Baptiste, 59253 La Gorgue (FR); LEFEVRE, Philippe, 59660 Haverskerque (FR)
(74) Mandataire: Plasseraud IP
(86) Numéro de dépôt international: PCT/FR2016/051820
(87) Numéro de publication internationale: WO 2017/013338

(56) Documents cités:
- US-A- 5 573 777
- US-A1- 2003 118 642
- US-A1- 2012 053 249
- US-B2- 6 998 481

## Description

La présente invention a pour objet des granules de mannitol utiles pour la préparation de comprimés par compression directe, qui ne requièrent qu'une faible quantité de lubrifiant pour être mis en oeuvre. La présente invention a également pour objet un procédé pour la préparation de tels granules. La présente invention englobe également des compositions pulvérulentes comprenant ces granules, ainsi que des procédés de compression directe mettant en œuvre ces compositions pulvérulentes. La présente invention porte enfin sur des comprimés préparés à partir de ces compositions pulvérulentes, ainsi que sur des comprimés à base de mannitol à faibles teneurs en lubrifiants.

### ART ANTERIEUR

Les techniques de compression directe permettent la fabrication de comprimés contenant des quantités précises d'ingrédients actifs, à vitesse rapide, et à coût relativement faible. Cette technologie consiste à compresser fortement une composition pulvérulente dans une matrice à l'aide d'un poinçon, de façon à lui donner la forme d'un comprimé. C'est la forte pression appliquée qui provoque une agrégation des particules de la poudre et produit un comprimé solide.

Ces compositions pulvérulentes comprennent typiquement des excipients et des ingrédients actifs d'intérêt, par exemple pharmaceutique, vétérinaire, cosmétique, alimentaire, nutraceutique, chimique ou agrochimique.

Les excipients les plus couramment rencontrés en compression directe sont les diluants, également appelés « excipients de compression directe », les lubrifiants, les (super-)désintégrants, les agents d'écoulement, les agents stabilisateurs de pH, les colorants, les arômes, les surfactants.

Afin d'être apte à former des comprimés, la composition pulvérulente à comprimer comprend toujours au minimum, un excipient de compression direct et un lubrifiant.

Les excipients de compression directe sont les composés majoritaires des comprimés et sont responsables des propriétés de comprimabilité et d'écoulement de la poudre. Les plus couramment utilisés sont la cellulose microcristalline et le lactose.

Le lubrifiant lui, permet l'éjection des matrices des comprimés néoformés. Il limite le stress induit par l'éjection et permet donc de préserver l'intégrité des comprimés. Le plus utilisé est le stéarate de magnésium, suivi du stéarate de calcium et du stéarylfumarate de sodium.

Lorsque les quantités de lubrifiant employées sont insuffisantes, il n'est plus possible d'obtenir des comprimés satisfaisants. Cela se traduit en particulier par l'apparition de clivage (« capping ») et/ou de traces de grippage.

Le clivage se présente sous la forme d'une fracturation horizontale des comprimés, soit en leur milieu soit au début d'une des deux parties bombées (phénomène appelé aussi décalotage). Le grippage correspond au collage d'une partie de la matière sur la matrice, collage qui perdure après éjection du comprimé. En dehors de l'augmentation de la force d'éjection mesurée (qui atteint alors typiquement 1000 N), le grippage est visible sur le comprimé : des raies verticales sont présentes et correspondent aux emplacements où le produit est resté collé sur la matrice.

Un comprimé satisfaisant ne doit pas présenter de traces de grippage, et a donc une tranche parfaitement lisse sur toute sa circonférence. Un tel comprimé doit par ailleurs avoir une surface homogène et lisse sur toute sa surface.

Pourtant indispensables, les lubrifiants présentent certains inconvénients. Un problème fréquemment rencontré avec les sels de stéarates est leur potentiel à ralentir la désintégration et la dissolution des comprimés. Un autre problème réside dans le goût particulièrement désagréable de ces lubrifiants, ainsi que dans leur coût relativement élevé.

Afin de pallier ce problème, récemment, dans sa demande de brevet US 2012/0053249 A1, la Demanderesse décrivait de façon intéressante un excipient multifonctionnel, capable de remplir le rôle d'excipient de compression directe, mais également de remplir partiellement le rôle de lubrifiant. Il s'agissait d'un coagglomérat de mannitol et d'amidon granulaire, qui permettait de réduire les quantités de stéarate de magnésium mises en oeuvre.

Un désavantage de ces granules réside dans la présence d'amidon granulaire, pourtant présentée comme essentielle dans cette précédente invention. En effet, l'amidon granulaire est facilement sujet à la contamination bactérienne, si bien qu'un contrôle étroit de la fabrication et du stockage est requis. L'amidon granulaire présente par ailleurs une teneur élevée en eau, qui peut nuire à la stabilité des ingrédients actifs des comprimés.

Le mannitol en revanche, utilisé seul, pourrait constituer un excellent excipient, notamment pour sa très grande inertie chimique vis-à-vis des principes actifs, et en raison de sa saveur et de la faible hygroscopicité de sa forme cristalline.

Il serait ainsi particulièrement avantageux de pouvoir s'abstenir de l'addition d'amidon granulaire dans les granules précédemment mis au point par la Demanderesse.

Le problème, déjà soulevé par la Demanderesse dans sa demande de brevet US 2012/0053249 A1 précitée, est que les poudres obtenues en utilisant moins de 0,5% d'amidon granulaire ne possédaient pas de propriétés satisfaisantes d'écoulement et de comprimabilité.

Après de nombreux travaux, la Demanderesse est parvenue à préparer des poudres de mannitol essentiellement exemptes d'amidon granulaire, qui non seulement se compriment et s'écoulent parfaitement, mais qui en plus sont douées de propriétés lubrifiantes.

Ces poudres sont des granules non sphériques de mannitol microcristallin, dans lesquelles le mannitol est à la fois sous forme cristalline α et sous forme cristalline β, le rapport α/β étant compris entre 10/90 et 90/10, qui présentent un diamètre volumique moyen D4,3 de 60 à 250 µm, une densité aérée d'au moins 465 g/L, et une surface spécifique supérieure à 0,75 m²/g.

En ce qui concerne la forme cristalline, la littérature reporte trois principales formes : les mannitols-α, -β et -δ. Pour des applications en compression directe, le mannitol-β est classiquement préféré, principalement en raison de sa stabilité. Par ailleurs, lorsque le mannitol est de façon analogue aux granules selon l'invention, sous la forme de particules non sphériques de structure microcristalline, certains auteurs décrivent comme néfaste la présence de mannitol-α. A titre d'exemple, le brevet US 6,998,481 B2 indique que la présence de mannitol-α nuit à la comprimabilité de la poudre.

Dans les coagglomérats de mannitol et d'amidon granulaire décrits dans la demande de brevet US 2012/0053249 A1 précitée, le mannitol était également partiellement sous forme a. La Demanderesse allait déjà alors à l'encontre d'un préjugé technique en ne préférant pas la forme purement β. Cependant, ce préjugé technique était vaincu au moyen d'une co-granulation avec de l'amidon granulaire. Il n'était donc pas du tout évident que des granules de mannitol privées d'amidon granulaire puissent également présenter un intérêt sous cette double forme cristalline.

La Demanderesse a réussi à développer ces granules grâce à un nouveau procédé de granulation par voie humide mis en oeuvre dans un atomiseur multiple-effets. Ce procédé est en particulier mis en oeuvre dans une tour d'atomisation dans laquelle l'angle formé par la paroi de la partie supérieure cylindrique et le toit de la chambre est égal à 90°. L'humidité relative en sortie de tour est choisie dans la gamme de 5 à 60 %.

Des poudres de mannitol pour compression directe ont déjà été décrites dans l'art antérieur. Cependant, aucune ne présentait la combinaison de caractéristiques des granules de l'invention.

Les poudres obtenues par les techniques de fusion / extrusion, par exemple telles que celles décrites dans les brevets US 4,661,647 A et US 5,160,680 A au nom de la Demanderesse, présentent une surface spécifique inférieure à 0,75 m²/g, et un diamètre moyen généralement supérieur à 300 µm. Ces poudres nécessitent des quantités supérieures de lubrifiant pour être mises en oeuvre, comparativement au granules de l'invention.

Les procédés d'atomisation simple-effet, par exemple tels que ceux décrits dans le document US 2011/0135927 A1, aboutissent à la formation de poudres constituées de particules sphériques, présentant un diamètre moyen généralement inférieur à 60 µm. Ces poudres ne sont pas viables sur le plan industriel, principalement parce qu'elles ne s'écoulent pas dans les presses utilisées en compression directe.

D'autres documents décrivent la préparation de granules non sphériques de mannitol microcristallin, mais dont la forme cristalline ne correspond pas à celle requise par la présente invention.

Des exemples de tels documents sont :
- le brevet US, 6,998,481 B2 (MERCK), qui porte sur des poudres de mannitol pour compression directe, dans lesquelles le mannitol est essentiellement sous forme cristalline β ;
- le brevet US 5,573,777 A au nom de la Demanderesse qui, bien que silencieux quant à la forme cristalline du mannitol, et comme il ressort de l'Exemple 1 ci-après, décrit des poudres dont le mannitol est en réalité essentiellement sous forme cristalline α.

Ces poudres nécessitent des quantités supérieures de lubrifiant pour être mises en oeuvre, comparativement aux granules de l'invention (voir l'Exemple 1).

### BUTS DE L'INVENTION

La présente invention a pour objectif de fournir des granules de mannitol essentiellement exempts d'amidon granulaire, qui nécessitent de faibles quantités de lubrifiant pour être mis en oeuvre.

La présente invention a pour objectif de répondre aux problèmes susmentionnés en proposant un excipient de mannitol qui possède par ailleurs les autres propriétés requises pour un excipient de compression directe, notamment en termes de comprimabilité et d'écoulement.

### RESUME DE L'INVENTION

Un premier objet de l'invention consiste en des granules non sphériques de mannitol microcristallin, caractérisés :
- en ce que ledit mannitol se présente à la fois sous forme cristalline α et sous forme cristalline β, le rapport α/β étant compris entre 10/90 et 90/10 ; et,
- en ce qu'ils sont essentiellement exempts d'amidon granulaire ; et,
- en ce qu'ils présentent un diamètre volumique moyen D4,3 de 60 à 250 µm ; et,
- en ce qu'ils présentent une densité aérée d'au moins 465 g/L ; et,
- en ce qu'ils présentent une surface spécifique supérieure à 0,75 m²/g.

Un second objet de l'invention consiste en un procédé de préparation de granules de mannitol consistant en :
- une étape (a) de préparation d'une solution de mannitol ;
- une étape (b) de séchage par atomisation de la solution de mannitol obtenue à l'étape (a) dans une tour d'atomisation multiple-effets (1), comprenant le recyclage des fines, caractérisée :
   ∘ en ce que l'humidité relative en sortie de tour est choisie dans une gamme allant de 5 à 60 % ; et,
   ∘ en ce la tour d'atomisation multiple-effets (1) comprend une chambre de base conique (2) constituée d'une partie inférieure cylindrique (2a) comprenant un lit fluidisé statique (3), d'une partie intermédiaire conique (2b), et d'une partie supérieure cylindrique (2c) ; l'angle (α_{Cy}) formé par la paroi de la partie supérieure cylindrique (2c) et le toit de la chambre (2) étant égal à 90° ;
- une étape (c) de récupération des granules ainsi obtenus.

Un troisième objet de l'invention consiste en une composition pulvérulente comprenant les granules de mannitol selon l'invention.

Un quatrième objet de l'invention consiste en un procédé de préparation de comprimés consistant en la compression directe d'une composition pulvérulente selon l'invention.

Un cinquième objet de l'invention consiste en un comprimé constitué de la composition pulvérulente selon l'invention, ou susceptible d'être obtenu, ou obtenu par le procédé de préparation de comprimés selon l'invention.

Un sixième objet de l'invention consiste en un comprimé essentiellement exempt d'amidon granulaire constitué :
- de 30,0 à 100,0 % de mannitol ;
- de moins de 0,8 % de lubrifiant ;
- de 0 à 70,0 % d'ingrédients autres que du mannitol et du lubrifiant ;
   les pourcentages étant exprimés en poids sec, et leur somme étant égale à 100 %.

Un septième objet de l'invention consiste en l'utilisation de granules conformes à l'invention en tant qu'excipient de compression directe.

### DESCRIPTION DETAILLEE DE L'INVENTION

Les granules selon l'invention permettent avantageusement la préparation industrielle de comprimés d'apparence et de dureté satisfaisante en présence de très faibles teneurs de lubrifiant.

Il en résulte qu'il est à présent possible de produire des comprimés à base de mannitol présentant de faibles teneurs en lubrifiant, qui présentent notamment un goût amélioré comparativement aux comprimés présentant des teneurs supérieures en lubrifiants.

Les granules de l'invention sont essentiellement exempts d'amidon granulaire, et permettent donc d'éviter les désavantages qui peuvent résulter de sa présence.

Ces granules peuvent être obtenus par un procédé particulier de séchage par atomisation dans une tour d'atomisation multiple-effets, comprenant le recyclage des fines, faisant également l'objet de la présente invention.

En plus de permettre la préparation de granules conformes à l'invention, ce procédé présente l'avantage d'être un procédé continu : la solution atomisée agglomère une poudre de mannitol microcristallin (« poudre primaire »), laquelle résulte elle-même du séchage par atomisation de ladite solution.

Le procédé de l'invention permet par ailleurs l'emploi de températures d'entrée et de sortie relativement basses, ce qui permet de modérer les dépenses énergétiques, tout en garantissant un procédé pertinent sur le plan industriel, en particulier en termes de productivité.

Un premier objet de l'invention consiste en des granules non sphériques de mannitol microcristallin, caractérisés :
- en ce que ledit mannitol se présente à la fois sous forme cristalline α et sous forme cristalline β, le rapport α/β étant compris entre 10/90 et 90/10 ; et,
- en ce qu'ils sont essentiellement exempts d'amidon granulaire ; et,
- en ce qu'ils présentent un diamètre volumique moyen D4,3 de 60 à 250 µm ; et,
- en ce qu'ils présentent une densité aérée d'au moins 465 g/L ; et,
- en ce qu'ils présentent une surface spécifique supérieure à 0,75 m²/g.

L'expression « granules non sphériques de mannitol microcristallin », est telle qu'elle est habituellement comprise de l'homme du métier. En particulier, au microscope électronique et à un grossissement de X100, ces granules sont généralement de formes variables, et de surface irrégulière. À un grossissement de X1000, de fines particules de fins cristaux agglomérés sont généralement visibles à leur surface. La qualification de granules « non sphériques » permet typiquement de les distinguer des particules obtenues par atomisation simple-effet d'une solution de mannitol, qui elles, sont qualifiées de « sphériques » par l'homme du métier. On entend par « structure microcristalline », une structure qui, observée au microscope électronique à un grossissement de X1000, ne présente en surface que des microcristaux et très rarement des cristaux de taille supérieures. Le microcristal est en particulier défini comme un cristal dont la somme de la longueur, de la largeur et de l'épaisseur est inférieure à 25 µm. Les particules et microcristaux présents peuvent présenter des formes très différentes, de formes arrondies à des formes allongées. Dans les granules de l'invention, cette microstructure n'est en particulier pas décrite comme « filamenteuse ».

En effet, même si des cristaux de formes aiguilles peuvent être présents, ceux-ci sont très largement minoritaires à la surface des granules. En comparaison, le brevet US 6,998,481 B2 présente une photo de granule de texture dite filamenteuse car seuls des microcristaux de forme aiguille sont visibles.

On note enfin que dans les granules de l'invention, les microcristaux ne sont généralement pas orientés.

Au microscope électronique et à un grossissement de X200, ces granules se différencient aisément des poudres de mannitol cristallines classiques, composées de macrocristaux bien individualisés, typiquement polyédriques, de surface régulière, d'épaisseur sensiblement constante mais de longueur et de largeur variables, généralement obtenues par simple cristallisation dans l'eau, à partir d'une solution sursaturée en mannitol. Ils se différencient encore des poudres de mannitol obtenues par agglomération d'une poudre primaire composée de macrocristaux de mannitol. Ces granules ne sont pas de structure microcristalline : les cristaux, bien que ne se présentant plus sous forme individualisée, sont encore bien visibles et apparaissent sous la forme d'arêtes vives dans ces granules. Ils se différencient également des poudres de mannitol obtenues par séchage par atomisation simple-effet (n'employant pas de lit fluidisé) d'une solution de mannitol, dont les particules, bien que composées de mannitol microcristallin, sont sphériques et de faible diamètre, généralement compris entre 10 et 50 µm. Ils se différencient encore des poudres de mannitol obtenues par fusion / extrusion, lesquelles sont formées de particules plus compactes et plus régulières, se présentant sous forme de pavés plus ou moins anguleux, et qui sont constituées de microcristaux généralement orientés.

Classiquement, pour que des poudres de mannitol puissent présenter l'aspect de granules non sphériques de structure microcristalline, ils sont préparés par agglomération d'une poudre primaire composée de particules de structure microcristalline.

Ainsi, alternativement, ou de façon complémentaire, les « granules non sphériques de mannitol microcristallin » peuvent être définis par le fait qu'ils sont obtenus, ou susceptibles d'être obtenus, par agglomération d'une poudre primaire de mannitol composée de particules de structure microcristalline, incluant préférentiellement le recyclage des fines.

Aussi, alternativement ou de façon complémentaire, les granules conformes à l'invention peuvent être définis par le fait qu'ils ne sont pas obtenus par atomisation simple-effet, par fusion / extrusion, ou par granulation de macrocristaux.

Les granules de l'invention sont aussi caractérisés par le fait que le mannitol se présente à la fois sous forme cristalline α et sous forme cristalline β, le rapport α/β étant compris entre 10/90 et 90/10.

La coexistence des deux formes cristallines α et β du mannitol, ainsi que leurs proportions, peut être déterminée par l'homme du métier par spectrométrie infrarouge ou par diffraction X, préférentiellement par spectrométrie infrarouge. On peut par exemple procéder selon la méthode telle que décrite dans l'Exemple 1 ci-après.

Préférentiellement, ce rapport en formes cristallines α/β est choisi dans la gamme allant de 15/85 à 85/15, préférentiellement de 20/80 à 80/20, préférentiellement de 25/75 à 75/25, préférentiellement de 30/70 à 70/30, préférentiellement de 35/65 à 65/35, préférentiellement de 40/60 à 60/40, par exemple de 45/55 à 55/45. Il est par exemple égal à 50/50.

Les granules de l'invention sont aussi caractérisés par le fait qu'ils sont essentiellement exempts d'amidon granulaire.

Par « essentiellement exempt d'amidon granulaire », il est entendu dans l'invention que le rapport en poids sec amidon granulaire / mannitol est inférieur à 0,5/99,5, préférentiellement inférieur à 0,4/99,6, préférentiellement inférieur à 0,3/99,7, préférentiellement inférieure à 0,2/99,8, préférentiellement inférieure à 0,1/99,9, voire de façon préférentielle, que les granules sont totalement exempts d'amidon granulaire.

De façon plus générale et avantageuse, ce qui est explicité ici s'applique à l'amidon, quelle que soit sa forme, par exemple à un amidon non-granulaire, fonctionnalisé ou non (éthérification, estérification), hydrolysé ou non.

Les granules de l'invention sont aussi caractérisés par le fait qu'ils présentent un diamètre volumique moyen D4,3 de 60 à 250 µm.

Ce diamètre volumique moyen D4,3 peut en particulier être déterminé par l'homme du métier au moyen d'un granulomètre à diffraction laser en voie sèche, par exemple selon la méthode telle que décrite dans l'Exemple 1 ci-après.

Le diamètre volumique moyen D4,3 des granules selon l'invention est généralement choisi dans la gamme allant de 60 à 240 µm, par exemple de 60 à 220 µm, voire de 60 à 200 µm. Ce diamètre volumique moyen D4,3 est préférentiellement supérieur à 80 µm, préférentiellement supérieur à 90 µm, préférentiellement encore supérieur à 100 µm.

Les granules de l'invention sont aussi caractérisés par le fait qu'ils présentent une densité aérée d'au moins 465 g/L.

La densité aérée des granules selon l'invention est généralement choisie dans la gamme allant de 465 à 600 g/L, par exemple de 465 à 580 g/L, voire de 465 à 550 g/L. Cette densité aérée est préférentiellement d'au moins 470 g/L, préférentiellement d'au moins 480 g/L, préférentiellement d'au moins 490 g/L, tout préférentiellement d'au moins 500 g/L.

Préférentiellement, les granules selon l'invention présentent par ailleurs une densité tassée d'au moins 470 g/L, généralement choisie dans la gamme allant de 470 à 700 g/L, préférentiellement de 500 à 650 g/L, tout préférentiellement de 550 à 600 g/L.

Cette densité aérée et cette densité tassée peuvent être déterminées par l'homme du métier par la méthode des éprouvettes graduées, en particulier selon la méthode préconisée par l'OMS (Document QAS/11.450 FINAL, 2012).

Les granules de l'invention sont aussi caractérisés par le fait qu'ils présentent une surface spécifique supérieure à 0,75 m²/g.

Cette surface spécifique peut être déterminée par l'homme du métier, par la méthode BET, par exemple selon l'a méthode telle que décrite dans l'Exemple 1 ci-après.

La surface spécifique des granules selon l'invention est généralement comprise entre 0,75 et 3,00 m²/g, par exemple comprise entre 0,75 et 2,50 m²/g, voire entre 0,75 et 2,00 m²/g, voire entre 0,75 et 1,50 m²/g, voire entre 0,75 et 1,20 m²/g. Cette surface spécifique est préférentiellement supérieure à 0,80 m²/g, préférentiellement supérieure à 0,85 m²/g, préférentiellement supérieure à 0,90 m²/g, préférentiellement supérieure à 0,95 m²/g, préférentiellement encore supérieure à 1,00 m²/g.

Les granules selon l'invention peuvent aussi être caractérisés en ce qu'il s'agit de granules pour compression directe ou de granules « directement compressibles ». On parle également classiquement d'« excipient de compression directe ». Les granules selon l'invention peuvent ainsi être comprimés directement, c'est-à-dire sans aucun traitement préalable de texturation ou de transformation physique, comme par exemple une étape préalable de granulation par voie sèche ou humide.

Il est entendu de l'homme du métier que cela signifie qu'ils sont aptes à former des comprimés de dureté suffisante, par compression directe, en la seule présence d'une quantité efficace de lubrifiant. Cette « quantité efficace » est telle qu'elle permet effectivement la formation de comprimés, c'est-à-dire typiquement, qu'il y a absence de clivage, de grippage, et que la force d'éjection est inférieure à 1000 Newtons, sur une production de 10 comprimés. Cette quantité efficace de lubrifiant n'excède généralement pas 3 % en poids sec, par rapport au poids sec total de la poudre à comprimer.

Cette aptitude à former des comprimés satisfaisant peut en particulier être déterminée par l'homme du métier par compression directe d'une composition pulvérulente constituée de l'excipient à tester et de lubrifiant, par exemple du stéarate de magnésium, en utilisant une force de compression de 10 kN, de manière à former des comprimés convexes de 10 mm de diamètre d'un rayon de courbure de 9 mm, et d'un poids de 400 mg. Les comprimés peuvent en particulier être formés au moyen d'une presse rotative, ou au moyen d'une presse de développement mono-poinçon qui simule la compression sur presse rotative industrielle, par exemple telle que celle utilisée dans les Exemples ci-après. Sur les comprimés ainsi obtenus, on procède à des mesures de dureté au moyen d'un duromètre, par exemple tel que celui utilisé dans l'Exemple 2 ci-après.

La dureté des comprimés préparés à partir de l'excipient à tester en la seule présence du lubrifiant, exprimée en Newtons (N), désigne ce que l'homme du métier appelle couramment « comprimabilité » de l'excipient.

Les granules selon l'invention peuvent donc également être caractérisés par leur comprimabilité, qui est généralement comprise entre 50 et 500 N, par exemple comprise entre 50 et 400 N, voire entre 50 et 300 N, voire entre 50 et 200 N, voire entre 50 et 150 N.

Cette comprimabilité est préférentiellement supérieure à 70 N, préférentiellement supérieure à 80 N, préférentiellement supérieure à 90 N, préférentiellement supérieure à 100 N.

Les granules selon l'invention peuvent également être caractérisés par leur note d'écoulement, laquelle est préférentiellement choisie dans la gamme allant de 3 à 15 secondes, préférentiellement de 4 à 10 secondes, tout préférentiellement de 4 à 8 secondes.

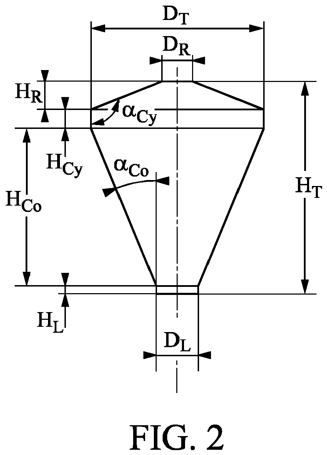

Cette note d'écoulement peut en particulier être déterminée par l'homme du métier selon la méthode préconisée par la pharmacopée européenne de référence « 2.9.16. Flowability, 01/2005: 20916 ; équipement selon la figure 2.9.16.-2».

Les granules selon l'invention peuvent également être caractérisés par leur friabilité, laquelle est généralement comprise entre 20 et 50 %, par exemple entre 30 et 50 %. Cette friabilité est préférentiellement inférieure à 40 %, par exemple comprise entre 20 et 40 %, par exemple entre 30 et 40 %.

Pour mesurer cette friabilité, l'homme du métier peut par exemple procéder selon la méthode telle que décrite dans l'Exemple 2 ci-après.

Les granules selon l'invention sont des « granules de mannitol », mais peuvent cependant comprendre d'autres ingrédients, en faibles quantités, et tant que cela ne contrevient pas aux propriétés recherchées dans la présente invention.

Des exemples d'autres ingrédients sont : des liants tels que la polyvinylpyrrolidone (PVP), la carboxyméthylcellulose (CMC), l'hydroxypropylméthylcellulose (HPMC), les dérives cellulosiques, la gomme acacia, la gélatine, les dérivés d'amidons tels que les maltodextrines, la gomme tragacanthe ; des minéraux ; des carbohydrates tels que des sucres et des polyols autres que du mannitol ; des additifs alimentaires, des colorants ; des ingrédients actifs nutraceutiques, pharmaceutiques, vétérinaires ou cosmétiques ; des conservateurs ; des agents stabilisants.

Préférentiellement, la teneur en autres ingrédients dans les granules, en particulier la teneur en carbohydrates autres que le mannitol, est inférieure à 15,0 %, préférentiellement inférieure à 10,0 %, préférentiellement inférieure à 5,0 %, préférentiellement inférieure à 2,0 %, préférentiellement inférieure à 1,0 %, préférentiellement encore inférieure à 0,5 % ; ces pourcentages étant exprimés en poids sec par rapport au poids total de matière sèche des granules. Tout préférentiellement, les granules conformes à l'invention sont exempts d'autres ingrédients. Dans ce dernier cas, cela signifie que les granules ne sont constitués que de mannitol et de ces impuretés.

Notons à cet égard que le mannitol utile à l'invention a préférentiellement une richesse en mannitol, en particulier en D-mannitol, supérieure à 95,0 % en poids sec, préférentiellement supérieure à 96,0 %, préférentiellement supérieure à 97,0 %, préférentiellement supérieure à 97,5 %, préférentiellement supérieure à 98,0 %, préférentiellement supérieure à 98,5 %, tout préférentiellement supérieure à 99,0 %.

Les autres composés du mannitol comprennent typiquement les substances liées au mannitol, en particulier le sorbitol, le maltitol et l'isomalt, les sucres réducteurs, le nickel, les métaux lourds. Leurs teneurs peuvent en particulier être déterminées par l'homme du métier selon les méthodes préconisées par la pharmacopée européenne, en particulier tirées du document de référence « Mannitol, 01/2014:0559 ».

En particulier, le mannitol utile à l'invention présente préférentiellement un taux de sucres réducteurs n'excédant pas 0,1 %, une teneur en nickel n'excédant pas 1 ppm et une teneur en métaux lourds n'excédant pas 5 ppm.

Les granules selon l'invention peuvent également être caractérisés par leur perte de masse à la dessiccation, laquelle est préférentiellement comprise entre 0,00 et 0,50 % en poids. Cette perte de masse à la dessiccation est préférentiellement inférieure à 0,40 % en poids, préférentiellement inférieure à 0,30 %, préférentiellement inférieure à 0,25 %, préférentiellement encore à 0,20 %, par exemple comprise entre 0,01 et 0,20%, voire entre 0,02 et 0,15 %.

Cette perte de masse à la dessiccation peut en particulier être déterminée par l'homme du métier selon la méthode préconisée par la pharmacopée européenne de référence « 2.2.32. Loss on drying, 07/2015 : 20232 ».

La présente invention a également pour objet un procédé de préparation de granules de mannitol, particulièrement utile pour la préparation de granules conformes à l'invention, qui consiste en :
- une étape (a) de préparation d'une solution de mannitol ;
- une étape (b) de séchage par atomisation de la solution de mannitol obtenue à l'étape (a) dans une tour d'atomisation multiple-effets (1), comprenant le recyclage des fines, caractérisée :
   ∘ en ce que l'humidité relative en sortie de tour est choisie dans une gamme allant de 5 à 60 % ;
   ∘ et en ce que la tour d'atomisation multiple-effets (1) comprend une chambre (2) constituée d'une partie inférieure cylindrique (2a) comprenant un lit fluidisé statique (3), d'une partie intermédiaire tronconique (2b), et d'une partie supérieure cylindrique (2c) ; l'angle (α_{Cy}) formé par la paroi latérale de la partie supérieure cylindrique (2c) et le toit de la chambre (2) étant égal à 90° ;
- une étape (c) de récupération des granules ainsi obtenus.

Préférentiellement, la solution de mannitol préparée à l'étape (a) présente une matière sèche en poids comprise entre 20 et 60 %, préférentiellement entre 30 et 50 %, préférentiellement entre 35 et 45 %, par exemple égale à 40 %.

Préférentiellement, cette solution est maintenue à une température telle qu'elle permet de conserver le mannitol à l'état dissous ou sous la forme de microcristaux.

Préférentiellement, l'humidité relative à laquelle l'étape (b) fait référence est choisie dans une gamme allant de 5 à 50 %, préférentiellement de 10 à 40 %, préférentiellement de 10 à 30 %, préférentiellement de 15 à 25 %.

Préférentiellement, la surface du toit de la chambre (2) de la tour d'atomisation (1) visée à l'étape (b) est essentiellement plat, préférentiellement totalement plat.

Préférentiellement, la chambre (2) de la tour d'atomisation (1) présente un volume de 8 à 1 500 m³, préférentiellement de 10 à 1000 m³, préférentiellement de 50 à 800 m³, préférentiellement de 100 à 700 m³, préférentiellement de 200 à 600 m³, préférentiellement de 300 à 500 m³. Ce volume est par exemple égal à 400 m³.

Préférentiellement, les dimensions de la tour d'atomisation (1) sont telles que le diamètre D_{T} est choisi dans la gamme allant de 2,0 à 16,0 m, préférentiellement de 5,0 à 15,0 m, préférentiellement de 8,0 à 12,0 m ; le rapport H_{T}/D_{T} est choisi dans la gamme allant de 0,9 à 1,6, préférentiellement de 0,9 à 1,4, préférentiellement de 1,0 à 1,2 ; le rapport D_{T}/D_{L} est choisi dans la gamme allant de 3,5 à 4,5 ; le rapport H_{Co} / H_{Cy} est choisi dans la gamme allant de 4,0 à 9,0, préférentiellement de 6,0 à 9,0, préférentiellement de 8,0 à 9,0 ; le rapport H_{Co} / H_{L} est choisi dans la gamme allant de 5,0 à 25,0, préférentiellement de 10,0 à 25,0, préférentiellement de 15,0 à 21,0 ; l'angle de cône α_{Co} est choisi dans la gamme allant de 18,0 à 25,0°, préférentiellement de 20,0 à 24,0°, préférentiellement de 22,0 à 23,0°.

Par exemple, les dimensions de la tour d'atomisation (1) sont telles que le diamètre D_{T} est égal à 10,0 m ; et/ou le rapport H_{T} / D_{T} est égal à 1,1 ; et/ou le rapport D_{T} / D_{L} est égal à 4,0 ; et/ou le rapport H_{Co} / H_{Cy} et égal à 8,3 ; et/ou le rapport H_{Co} / H_{L} est égal à 18,2 ; et/ou l'angle α_{Co} est égal à 22,4°.

Préférentiellement le débit est choisi de façon à ce que la vitesse de l'air du lit fluidisé statique soit de 0,3 à 1,5 m/s.

Pour le recyclage des fines, les fines et l'air sont préférentiellement extraits au niveau du toit de la chambre (2) de la tour d'atomisation (1), et les fines sont préférentiellement recyclées en bas de tour, i.e. dans la portion inférieure de la partie intermédiaire (2b) de la chambre (2).

L'extraction des fines peut avantageusement se faire par le biais de deux cheminées d'extraction (4a et 4b), avantageusement opposées par rapport à l'axe vertical de la tour d'atomisation (1). Leur bord est avantageusement situé à au moins 1 m de la paroi de la partie cylindrique (2c).

Avantageusement, les fines et l'air extraits de la chambre de la tour sont séparés au moyen d'au moins un cyclone, préférentiellement deux cyclones en série.

La pression de buse de pulvérisation est préférentiellement choisie dans la gamme allant de 30 à 300 bars, préférentiellement de 150 à 250 bars, pour un débit de solution allant de 1000 à 7000 kg/h, préférentiellement de 3000 à 5000 kg/h, par exemple de 4000 à 4200 kg/h.

Préférentiellement, la température de l'air d'entrée en amont de la tête de tour (« température d'entrée ») est choisie dans la gamme allant de 120 à 240°C, préférentiellement de 170 à 210°C. Cette température est par exemple égale à 190°C.

Préférentiellement, la température de l'air en sortie de tour (« température de sortie ») est comprise entre 50 et 120°C, préférentiellement entre 50 et 100°C, préférentiellement entre 50 et 90°C. Cette température de sortie est préférentiellement choisie dans la gamme allant de 60 à 80°C.

Préférentiellement, la température d'air du lit fluidisé statique (3) est choisie dans la gamme allant de 50 à 120°C, préférentiellement de 90 à 120°C. Cette température est par exemple égale à 110°C.

Préférentiellement, le procédé selon l'invention comprend en outre une étape (b'), antérieure à l'étape (c), de refroidissement des granules néoformés, préférentiellement au moyen d'un passage sur lit fluidisé vibré, à une température préférentiellement comprise entre 15 et 25°C, par exemple à une température de 20°C.

Dans le cas où les granules de l'invention comprendraient d'autres ingrédients que le mannitol, le procédé selon l'invention inclut la mise en oeuvre de ces autres ingrédients, qui peuvent être introduit sous forme sèche dans la chambre d'atomisation, par exemple *via* le système de recyclage des fines ou d'une entrée additionnelle, et/ou sous forme de suspension et/ou de solution, par exemple *via* la solution de mannitol pulvérisée.

La présente invention a également pour objet une composition pulvérulente, en particulier une composition pulvérulente pour compression directe, comprenant les granules de mannitol selon l'invention.

Cette composition pulvérulente est préférentiellement constituée de :
- 30,0 à 100,0 % de granules de mannitol selon l'invention ;
- 0,0 à 70,0 % d'ingrédients autres que les granules de mannitol selon l'invention ;
   les pourcentages étant exprimés en poids sec, et leur somme étant égale à 100 %.

Des exemples d'ingrédients autres sont typiquement :
- des excipients de compression directe ou des diluants autres que le mannitol de l'invention, par exemple (i) des polyols directement compressibles tels que les formes directement compressible du sorbitol, maltitol, xylitol, isomalt, lactitol, erythritol, ou du mannitol autre que celui selon l'invention (ii) des sucres directement compressible tels que les formes directement compressible du saccharose, dextrose, dextrates, lactose, allulose (iii) de la cellulose microcristalline, (iv) des minéraux directement compressibles ;
- des lubrifiants ;
- des dispersants ou des désintégrants, tels que le glycolate d'amidon sodique, la carboxymethylcellulose réticulée, la polyvinylpyrrolidone réticulée, les amidons ;
- des agents de granulation tels que la polyvinylpyrrolidone, la gomme acacia, le dextrose, la gélatine, les maltodextrines, les amidons, les dérivés d'amidons, la gomme tragacanthe ;
- des additifs alimentaires, tels que les arômes, les acidifiants ;
- des colorants tels que les colorants minéraux, les pigments ou les colorants solubles ;
- des agents de glissement (par exemple le dioxyde de silice), ou des agents anti-adhérents (par exemple le talc) ;
- des ingrédients actifs, notamment pharmaceutiques, vétérinaires, nutraceutiques, ou cosmétiques.

Préférentiellement, la composition pulvérulente pour compression directe conforme à l'invention présente une teneur en granules de mannitol selon l'invention d'au moins 40,0 %, préférentiellement d'au moins 50,0 %, préférentiellement d'au moins 60,0 %, préférentiellement d'au moins 70,0 %, préférentiellement d'au moins 80,0 %, préférentiellement d'au moins 90,0 % ; ces pourcentages étant exprimés en poids sec de granules conformes à l'invention par rapport au poids sec total de la composition pulvérulente. Cette teneur en granules de mannitol est généralement inférieure à 100,0 %. Elle est typiquement choisie dans la gamme allant de 90,0 à 99,0 %, voire de 95,0 à 99,0 %.

Préférentiellement, la composition selon l'invention présente une teneur en lubrifiants de 0,1 à 3,0 %, généralement de 0,2 à 3,0 %, voire de 0,3 à 3,0 %, voire de 0,4 à 3,0 %, voire de 0,5 à 3,0 %, voire de 0,6 à 3,0 % ; ces pourcentages étant exprimés en poids sec de lubrifiants par rapport au poids sec total de la composition pulvérulente. Préférentiellement, cette teneur en lubrifiant est inférieure à 2,5 %, préférentiellement inférieure à 2,0 %, préférentiellement inférieure à 1,5%, préférentiellement inférieure à 1,0 %. Elle est par exemple choisie dans la gamme allant de 0,6 à 0,9 %, voire de 0,6 à 0,8 %, voire de 0,6 à 0,7 %.

Préférentiellement, les lubrifiants de l'invention comprennent le stéarate de magnésium, à une teneur préférentiellement supérieure à 30,0 %, préférentiellement supérieure à 50,0 %, préférentiellement supérieure à 90,0 % ; ces pourcentages étant exprimés en poids sec de stéarate de magnésium par rapport au poids sec total des lubrifiants. Tout préférentiellement, le lubrifiant de l'invention est entièrement constitué de stéarate de magnésium.

Préférentiellement, dans la composition pulvérulente conforme à l'invention, les granules de mannitol selon l'invention représentent au moins 50,0 % des excipients de compression directe, préférentiellement au moins 60,0 %, préférentiellement au moins 70,0 %, préférentiellement au moins 80,0 %, préférentiellement au moins 90,0 %, préférentiellement au moins 95,0 %, préférentiellement au moins 98,0 %, préférentiellement au moins 99,0 % ; ces pourcentages étant exprimés en poids sec de granules conforme à l'invention par rapport au poids sec total d'excipients de compression de la composition pulvérulente. Tout préférentiellement, la composition pulvérulente est exempte d'excipients de compression directe autres que les granules de mannitol conformes à l'invention.

La présente invention a également pour objet un procédé de préparation de comprimés, préférentiellement pour diminuer les quantités de lubrifiant dans ces comprimés, consistant en la compression directe d'une composition pulvérulente selon l'invention, préférentiellement au moyen d'une presse rotative.

La présente invention a également pour objet un comprimé composé de la composition pulvérulente conforme à l'invention, ou susceptible d'être obtenu par, ou obtenu par le procédé de préparation de comprimés par compression directe selon l'invention.

On entend par « comprimé » au sens de la présente invention, une préparation solide obtenue par compression directe d'une composition pulvérulente. Le comprimé peut être par exemple à destination alimentaire, pharmaceutique, cosmétique, nutraceutique. Il peut s'agir de comprimés à sucer, à croquer, à avaler, de comprimés orodispersibles, de comprimés effervescents. Ces comprimés peuvent être destinés à l'humain, adulte ou enfant, ou à l'animal. Il peut également s'agir de comprimés à destination chimique ou agrochimique. Ces comprimés peuvent être simple-couche ou multicouches. Dans la présente invention, les comprimés sont préférentiellement de forme convexe.

La présente invention a également pour objet un comprimé essentiellement exempt d'amidon granulaire constitué :
- de 30,0 à 100,0 % de mannitol ;
- de moins de 0,8 % de lubrifiant ;
- de 0 à 70,0 % d'ingrédients autres que du mannitol et du lubrifiant ;
   les pourcentages étant exprimés en poids sec, et leur somme étant égale à 100 %.

Préférentiellement, le comprimé selon l'invention présente une teneur en mannitol d'au moins 40,0 %, préférentiellement d'au moins 50,0 %, préférentiellement d'au moins 60,0 %, préférentiellement d'au moins 70,0 %, préférentiellement d'au moins 80,0 %, préférentiellement d'au moins 90,0 % ; ces pourcentages étant exprimés en poids sec de mannitol par rapport au poids sec total du comprimé. Cette teneur en mannitol est généralement inférieure à 100,0 %. Elle est typiquement choisie dans la gamme allant de 90,0 à 99,0 %, voire de 95,0 à 99,0 %.

Préférentiellement, le comprimé selon l'invention présente une teneur en lubrifiants de 0,1 à 3,0 %, généralement de 0,2 à 3,0 %, voire de 0,3 à 3,0 %, voire de 0,4 à 3,0 %, voire de 0,5 à 3,0 %, voire de 0,6 à 3,0 % ; ces pourcentages étant exprimés en poids sec de lubrifiants par rapport au poids sec total du comprimé. Préférentiellement, cette teneur en lubrifiant est inférieure à 2,5 %, préférentiellement inférieure à 2,0 %, préférentiellement inférieure à 1,5 %, préférentiellement inférieure à 1,0 %. Elle est par exemple choisie dans la gamme allant de 0,6 à 0,9 %, voire de 0,6 à 0,8 %, voire de 0,6 à 0,7 %.

Préférentiellement, le mannitol des comprimés se présente à la fois sous forme cristalline α et sous forme cristalline β, le rapport en α/β étant notamment tel que défini avant.

La présente invention a également pour objet l'utilisation des granules conformes à l'invention comme excipient de compression directe.

Préférentiellement, les granules conformes à l'invention sont en outre utilisés pour diminuer les quantités de lubrifiant dans une composition pulvérulente pour compression directe.

Notons que dans la présente invention, il est entendu que l'expression « compris entre X et Y » couvre une plage de valeurs excluant les bornes citées, tandis que l'expression « dans la gamme allant de X à Y » ou bien « de X à Y » couvre une plage de valeurs incluant les bornes citées.

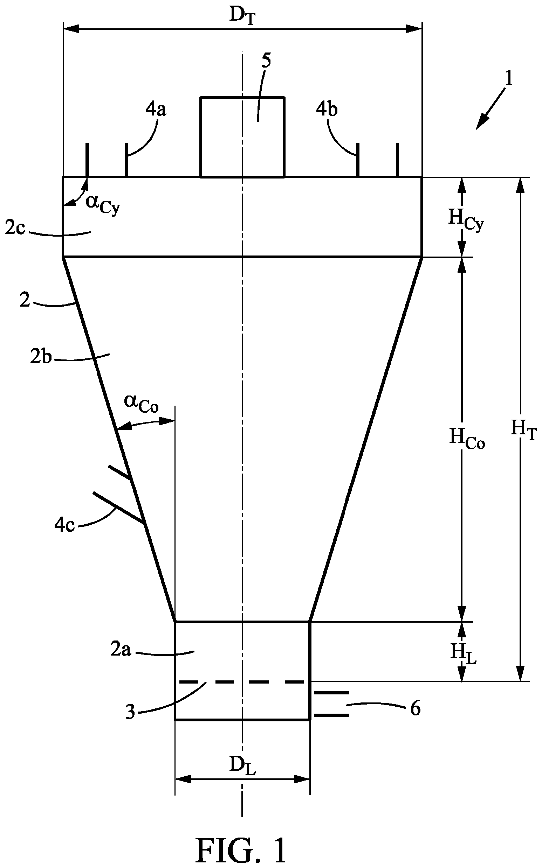

La **Figure 1** est une représentation schématique d'une tour d'atomisation multiple-effets, comprenant un dispositif pour le recyclage des fines, utile à l'invention. La tour d'atomisation 1 comprend une chambre 2 de hauteur H_{T} constituée d'une partie inférieure cylindrique 2a de hauteur H_{L}, comprenant un lit fluidisé statique 3, d'une partie intermédiaire tronconique 2b de hauteur H_{C0}, de plus petit diamètre D_{L}, de plus grand diamètre D_{T} et d'angle de cône α_{Co}, et d'une partie supérieure cylindrique 2c de hauteur H_{Cy} et de diamètre D_{T}. L'angle α_{Cy} formé par la paroi latérale de la partie supérieure cylindrique 2c et le toit de la chambre 2 est égal à 90°. Pour le recyclage des fines, la tour d'atomisation comprend deux cheminées d'extractions opposées 4a et 4b situées au niveau du toit de la chambre 2, et une entrée 4c pour les fines située dans la portion inférieure de la partie intermédiaire tronconique 2b de la chambre 2. La tour inclut un dispositif 5 qui comprend un système de pulvérisation et un disperseur d'air. La tour inclut une entrée d'air 6 qui alimente le lit fluidisé statique 3.

La **Figure 2** est une représentation schématique d'une chambre pour une tour d'atomisation multiple-effets, comprenant un dispositif pour le recyclage des fines, comprenant les mêmes caractéristiques que la chambre 2 de l'exemple de la Figure 1 à l'exception des caractéristiques suivantes : l'angle α_{Cy} formé par la paroi latérale de la partie supérieure cylindrique et le toit de la chambre est égal à 110°, comme pour l'atomiseur MSD 20 (NIRO) utilisé dans la demande de brevet US 2012/0053249 A1 précitée.

L'invention sera mieux comprise à l'aide des exemples qui suivent, lesquels se veulent illustratifs et non limitatifs.

### EXEMPLES

### 1. Excipients base mannitol testés

Plusieurs poudres de mannitol granulé ont été préparées par séchage par atomisation d'une solution de mannitol à 40 % de matière sèche dans une tour d'atomisation multiple-effets (du type MSD, NIRO) comprenant le recyclage des fines en bas de tour. Le débit d'air en amont était de 34000 Nm³/h, et le débit d'air du lit fluidisé statique de 15000 Nm³/h. La température d'air du lit fluidisé statique était de 110°C. Le débit de la solution atomisée était d'environ 4200 kg/h. Pour atomiser la solution, deux buses haute-pression 645 x 32 (SPRAYING SYSTEM SB) ont été utilisées.

Pour la réalisation des granules de mannitol IN-1, IN-2, et CP-1 à CP-4, une tour d'atomisation conforme à l'invention dotée d'une chambre 2 telle que représentée en Figure 1, i.e. ayant les dimensions suivantes a été utilisée :
- H_{T} = 10,7 m
- H_{Cy} = 1,1 m
- H_{Co} = 9,1 m
- H_{L} = 0,5 m
- D_{T} = 10 m
- D_{L} = 2,5 m
- α_{Cy} = 90°
- α_{Co} = 22,4°
- volume = 400 m³.

Pour la réalisation des granules de mannitol CP-5 et CP-6, une tour d'atomisation dotée d'une chambre telle que représentée en Figure 2, i.e. ayant un angle α_{Cy} de 110°, a été utilisée. Cette chambre d'atomisation correspond à celle utilisée dans la demande de brevet US 2012/0053249 A1 précitée. Les dimensions de cette chambre sont les suivantes :
- H_{T} = 12,3 m
- H_{Cy} = 1,1 m
- H_{Co} = 9,1 m
- H_{L} = 0,5 m
- D_{T} = 10 m
- D_{L} = 2,5 m
- D_{R} = 1,7 m
- H_{R} = 1,6 m
- α_{Cy} = 110°
- α_{Co} = 22,4°
- volume = 450 m³.

Les températures de sortie, les températures d'entrée et les humidités relatives de sortie qui ont été utilisées sont indiquées dans le Tableau 1.

Les granules AA-1 correspondent au produit PARTECK® M200 (MERCK), décrit dans le brevet US 6,998,481 B2.

Les granules AA-2 correspondent au produit PEARLITOL® 400DC (ROQUETTE FRERES), décrit dans le brevet US 5,160,680 A.

Les granules AA-3 correspondent au produit PEARLITOL® 200SD (ROQUETTE FRERES), décrit dans le brevet US 5,573,777 A à l'Exemple 1.

Les granules AA-4 correspondent au produit MANNOGEM® EZ (SPI PHARMA).

### 2. Compression directe

Dans les exemples qui suivent, les poudres ont été comprimées en utilisant une force de compression de 10 kN, de manière à former des comprimés convexes de 10 mm de diamètre d'un rayon de courbure de 9 mm, et d'un poids de 400 mg.

La presse employée était une presse de développement mono-poinçon qui simule la compression sur presse rotative industrielle (STYLCAM® 200R, MEDEL'PHARM), pilotée à l'aide du logiciel ANALIS, en utilisant le profil standard de la STYLCAM®. Cette presse permet avantageusement de simuler le fonctionnement des presses rotatives industrielles. La presse était réglée à une vitesse de 10 comprimés par minute.

Avant l'étape de compression, les poudres ont été mélangées intimement au lubrifiant. Ce mélange a été réalisé plus précisément en plaçant la poudre et le lubrifiant dans un contenant étanche et de volume compris entre 2 et 3 fois le volume de poudre à tester. Ce contenant a ensuite été fixé dans un mélangeur épicycloïdal (TURBULA T2C, Willy A. Bachofen AG Maschinenfabrik, CH-4005 Basel), réglé à environ 49 rotations par minute, et ledit mélangeur a été mis en rotation pendant 5 minutes.

### EXEMPLE 1 - EVALUATION DES QUANTITES MINIMUM DE LUBRIFIANTS REQUISES POUR COMPRIMER DIFFERENTS EXCIPIENTS DE MANNITOL

Dans cet exemple, les quantités minimum de lubrifiants requises pour comprimer les différents excipients de mannitol ont été déterminées.

Le lubrifiant employé était le stéarate de magnésium (stéarate de magnésium Bärlocher végétal, WIGA PHARMA GmbH).

Les poudres ont par ailleurs été caractérisées du point de vue de leur polymorphisme cristallin (rapport en forme cristalline α/β), de leur diamètre volumique moyen D4,3, de leur densité aérée et de leur surface spécifique.

Les résultats obtenus sont présentés dans le Tableau 1. Afin de faciliter la lecture, la 1^{ère} colonne indique si les granules sont destinés à illustrer l'invention (« IN-X »), s'il s'agit de granules comparatifs (« CP-X »), ou s'il s'agit de granules illustrant l'art antérieur (« AA-X »).
1. Détermination de la densité aérée. Les densités aérées des excipients à tester ont été mesurée selon la méthode préconisée par l'OMS (Document QAS/11.450 FINAL, 2012).
2. Détermination de la surface spécifique. Les surfaces spécifiques des excipients à tester ont été déterminées grâce à un analyseur de surface spécifique (BECKMAN-COULTER, de type SA3100), basé sur un test d'absorption de l'azote sur la surface du produit soumis à l'analyse, en suivant la technique décrite dans l'article BET Surface Area by Nitrogen Absorption de S. BRUNAUER et al. (Journal of American Chemical Society, 60, 309, 1938). L'analyse BET a été réalisée en 3 points.
3. Détermination du rapport α/β. Le rapport en formes cristallines α/β a été déterminé par spectrométrie infrarouge à transformée de Fourier, par la méthode du pastillage au bromure de potassium.
   Les échantillons ont été analysés par la technique de pastillage au bromure de potassium sur un spectromètre (Nexus™ FT-IR, NICOLET), équipé du logiciel OMNIC (Thermo Electron Corporation). La calibration et la quantification a été réalisée à l'aide du logiciel TQ-Analyst (Thermo Electron Corporation), à partir des régions spectrales (3050-2850 cm⁻¹) enregistrés avec un nombre de balayages de 20, et une résolution de 4 cm⁻¹.
   La calibration a été réalisée au moyen des formes α et β identifiées comme telles grâce à la description faite par L. Walter-Levy (Walter-Levy L. (23/12/1968). Cristallochimie. Sur les variétés cristallines du D-mannitol. C.R. Acad. Sc., Paris, t. 267, Série C, 1779-82), et de 5 mélanges physiques de ces deux formes, couvrant la gamme (85, 65, 50, 35, et 15 %). Ces mélanges, et les formes pures, ont été broyés dans un mortier en agate avant analyse. La quantification a été obtenue en utilisant la calibration précédente.
4. Détermination du diamètre moyen. Le diamètre moyen des excipients à tester a été mesuré par diffraction laser, au moyen d'un dispositif LS 13-320 (BECKMAN-COULTER), équipé de son module de dispersion poudre (voie sèche), en suivant le manuel technique et les spécifications du constructeur. Les conditions opératoires de vitesse de vis sous trémie et d'intensité de vibration de la goulotte de dispersion ont été déterminées de manière à ce que la concentration optique soit comprise entre 4 % et 12 %, idéalement de 8 %. La gamme de mesure du granulomètre à diffraction était de 0,04 µm à 2000 µm. Les résultats ont été calculés en % volumique, et exprimés en µm. La valeur du diamètre moyen volumique (moyenne arithmétique) D4,3 a été déterminée à partir de la courbe de distribution granulométrique.
5. Détermination de la quantité minimum de lubrifiant nécessaire pour la préparation de comprimés. Afin de déterminer la quantité minimale de lubrifiant nécessaire pour la préparation de comprimés, des compositions pulvérulentes utilisant des quantités croissantes de lubrifiant ont été comprimées par compression directe, comme indiqué au point 2, en introduction des exemples. Pour chaque essai 10 comprimés ont été préparés. La quantité minimale de lubrifiant est celle pour laquelle : (i) Il n'apparait aucun grippage ni aucun clivage, et (ii) la force d'éjection mesurée est inférieure à 1000 N.

Les résultats obtenus sont présentés dans le Tableau 1.

**Tableau 1 :**

| | Procédé | | | | Caractéristiques des granules | | | | Lubrification |
|---|---|---|---|---|---|---|---|---|---|
| Ref | Température d'entrée | Température de sorite | Hymidité relative | α_{Cy} | Rapport α/β | Densité aérée | Diam. Vol. Moy. D4,3 | Surface spécifique | % min de lubrifiant |
| IN-1 | 190°C | 69°C | 20,3 % | 90° | 50/50 | 510 g/L | 163 µm | 1,10 m²/g | 0,6 % |
| IN-2 | 190°C | 71°C | 18,9 % | 90° | 50/50 | 517 g/L | 172 µm | 1,25 m²/g | 0,6 % |
| CP-1 | 140°C | 48°C | 55,0 % | 110° | 55/45 | 403 g/L | 168 µm | 2,15 m²/g | 1,0 % |
| CP-2 | 160°C | 52°C | 45,2 % | 110° | 60/40 | 426 g/L | 175 µm | 2,10 m²/g | 1,0 % |
| CP-3 | 180°C | 62°C | 28,2 % | 110° | 60/40 | 444 g/L | 155 µm | 2,00 m²/g | 1,0 % |
| CP-4 | 220°C | 79,5°C | 13,3 % | 110° | 60/40 | 465 g/L | 151 µm | 0,75 m²/g | 1,0 % |
| CP-5 | 310°C | 125°C | 2,6 % | 90° | 90/10 | 500 g/L | 172 µm | 0,20 m²/g | 2,5% |
| CP-6 | 130°C | 44,8°C | 62,5 % | 90° | 40/60 | 438 g/L | 221 µm | 2,00 m²/g | 1,5 % |
| AA-1 | Procédé selon le brevet US 6,998,481 B2 | | | | < 10/90 | 559 g/L | 197 µm | 3,60 m²/g | 1,2 % |
| AA-2 | Procédé selon le brevet US 5,160,680 A | | | | 15/90 | 682 g/L | 449 µm | 0,40 m²/g | 0,8 % |
| AA-3 | Procédé selon le brevet US 5,573,777 A | | | | > 90/10 | 509 g/L | 157 µm | 0,45 m²/g | 2,0 % |
| AA-4 | / | | | | > 90/10 | 450 g/L | 120 µm | 1,20 m²/g | 2,0 % |

Les granules comparatifs CP-1 à CP-6 et les granules de l'art antérieur AA-1 à AA-4 nécessitent des quantités de lubrifiants de 0,8 à 2,5 % pour la confection de comprimés. Ces granules présentent en effet une densité aérée insuffisante et/ou ou une surface spécifique insuffisante, et/ou une forme cristalline non conforme à l'invention.

Les résultats obtenus avec les granules CP-1 à CP-4 permettent en particulier de montrer que le simple retrait de l'amidon dans les procédés employés dans la demande de brevet US 2012/0053249 A1 précitée, ne permet pas d'obtenir des granules ayant la combinaison de caractéristiques des granules de l'invention, et donc de répondre au problème technique de l'invention.

Les granules de l'invention IN-1 et IN-2 nécessitent des quantités moindres de lubrifiant, et permettent donc la réduction des teneurs en lubrifiant dans les comprimés, qui présentent alors notamment, un goût amélioré.

### EXEMPLE 2 - CARACTERISTIQUES DE GRANULES SELON L'INVENTION

Dans cet exemple, d'autres caractéristiques des granules IN-1 et IN-2 conformes à l'invention ont été déterminées. Les résultats obtenus sont présentés dans le Tableau 2.
1. Détermination de la comprimabilité. Des compositions pulvérulentes constituées des granules IN-1 ou IN-2 et de stéarate de magnésium (stéarate de magnésium Bärlocher végétal, WIGA PHARMA GmbH), ont été comprimées par compression directe. La dureté des comprimés a été mesurée à l'aide d'un duromètre (SCHLEUNIGER PHARMATRON 8M). Cette dureté, correspond à la comprimabilité de l'excipient.
2. Détermination de la note d'écoulement. La noté d'écoulement a été déterminée selon la méthode préconisée par la pharmacopée européenne de référence « 2.9.16. Flowability, 01/2005: 20916 ; équipement selon la figure 2.9.16.-2».
3. Détermination de la friabilité. Une poudre de granules conformes à l'invention a été soumise à une action mécanique dans un friabilimètre (ERWEKA TAR220, ERWEKA, 63150 Heusenstamm) équipé d'un tambour d'abrasion (choisi dans les références de pièces ERWEKA: ref 11-3x5-3xxx ou ref 11-3x5-x3xx ou ref 11-3x5-4xxx ou ref 11-3x5-x4xx), tournant à une vitesse de rotation uniforme de 25 tours par minute, dans lequel 5 billes d'acier identiques, d'un diamètre de 17 mm et d'un poids de 18,87 g ont été introduites. 15 g de la poudre ayant une granulométrie comprise entre 100 à 200 µm ont été introduits dans la chambre de concassage. L'appareil a été mis en rotation 15 minutes. La proportion pondérale représentée par le résidu retenu sur un tamis d'une largeur de maille de 100 µm a été déterminée. La valeur de la friabilité correspond au pourcentage de poudre non retenu par le tamis. La friabilité est d'autant plus grande que le pourcentage en poudre non retenu par le susdit tamis est grand.
4. Détermination de la perte de masse à la dessiccation. La perte de masse à la dessiccation a été déterminée selon la méthode préconisée par la pharmacopée européenne se référence « 2.2.32. Loss on drying, 07/2015 : 20232 ».
5. Détermination de la richesse en D-mannitol. La richesse en D-mannitol a été déterminée selon la méthode préconisée par la pharmacopée européenne dans le document de référence « Mannitol, 01/2014:0559 ».
6. Détermination de la densité tassée. La densité tassée a été mesurée selon la méthode préconisée par l'OMS (Document QAS/11.450 FINAL, 2012).

**Tableau 2**

| **Granules** | **IN-1** | **IN-2** |
|---|---|---|
| Comprimabilité | 107 N | 112 N |
| Note d'écoulement | 5 secondes | 6 secondes |
| Friabilité | 37,6 % | 39,6 % |
| Perte de masse à la dessiccation | 0,07 % | 0,10 % |
| Richesse en D-mannitol | 98,5 % | 98,9 % |
| Densité tassée | 560 g/L | 575 g/L |

Les granules selon l'invention possèdent les qualités requises pour un excipient de compression destiné à une production industrielle de comprimés. Ils permettent un remplissage correct des matrices, i.e. un remplissage uniforme et reproductible d'une quantité précise de poudre, et s'écoulent correctement dans les équipements utilisés en compression directe. Ils sont stables chimiquement et physiquement. Ils sont suffisamment cohésifs pour permettre leur transport ou la réalisation de mélanges. Ils ne gênent pas la biodisponibilité des autres ingrédients de la poudre et permettent l'obtention de comprimés qui se dissolvent correctement, en particulier au contact de l'eau. Ils permettent le mélange homogène des ingrédients de la composition, et possèdent une bonne capacité d'absorption. Ils permettent la confection de comprimés de texture et de goût acceptables, qui sont nécessaires lorsque le comprimé est destiné à être ingéré. Ils engendrent des coûts de conditionnement et de transport qui respectent les standards du commerce, c'est-à-dire qu'il y a un bon rapport entre la masse de poudre transportée et le volume nécessaire pour conditionner cette masse.

## Revendications

1. Granules non sphériques de mannitol microcristallin, **caractérisés :**
- **en ce que** ledit mannitol se présente à la fois sous forme cristalline α et sous forme cristalline β, le rapport α/β étant compris entre 10/90 et 90/10 ; et,
- **en ce qu'**ils sont essentiellement exempts d'amidon granulaire ; et,
- **en ce qu'**ils présentent un diamètre volumique moyen D4,3 de 60 à 250 µm ; et,
- **en ce qu'**ils présentent une densité aérée d'au moins 465 g/L ; et,
- **en ce qu'**ils présentent une surface spécifique supérieure à 0,75 m²/g.

2. Granules selon la revendication 1, **caractérisés en ce que** le rapport α/β dudit mannitol est de 15/85 à 85/15.

3. Granules selon l'une ou l'autre des revendications 1 ou 2, **caractérisés en ce qu'**ils présentent un diamètre volumique moyen D4,3 supérieur à 80 µm.

4. Granules selon l'une quelconque des revendications 1 à 3, **caractérisés en ce qu'**ils présentent une densité aérée d'au moins 470 g/L.

5. Granules selon l'une quelconque des revendications 1 à 4, **caractérisés en ce qu'**ils présentent une surface spécifique supérieure à 0,80 m²/g.

6. Granules selon l'une quelconque des revendications 1 à 5, **caractérisés en ce qu'**ils présentent une comprimabilité comprise entre 50 et 500 N.

7. Granules selon l'une quelconque des revendications 1 à 6, **caractérisés en ce qu'**ils présentent une note d'écoulement de 3 à 15 secondes.

8. Procédé de préparation de granules de mannitol tels que définis dans l'une quelconque des revendications 1 à 7, consistant en :
- une étape (a) de préparation d'une solution de mannitol ;
- une étape (b) de séchage par atomisation de la solution de mannitol obtenue à l'étape (a) dans une tour d'atomisation multiple-effets (1), comprenant le recyclage des fines, caractérisée :
∘ en ce que l'humidité relative en sortie de tour est choisie dans une gamme allant de 5 à 60 % ; et,
∘ en ce que la tour d'atomisation multiple-effets (1) comprend une chambre (2) constituée d'une partie inférieure cylindrique (2a) comprenant un lit fluidisé statique (3), d'une partie intermédiaire tronconique (2b), et d'une partie supérieure cylindrique (2c) ; l'angle (α_{Cy}) formé par la paroi latérale de la partie supérieure cylindrique (2c) et le toit de la chambre (2) étant égal à 90° ;
- une étape (c) de récupération des granules ainsi obtenus.

9. Composition pulvérulente comprenant des granules tels que définis dans l'une quelconque des revendications 1 à 7.

10. Procédé de préparation de comprimés consistant en la compression directe d'une composition pulvérulente selon la revendication 9.

11. Comprimé constitué de la composition pulvérulente telle que définie dans la revendication 9, ou susceptible d'être obtenu par, ou obtenu par le procédé telle que défini dans la revendication 10.

12. Comprimé essentiellement exempt d'amidon granulaire constitué :
- de 30,0 à 100,0 % de mannitol ;
- de moins de 0,8 % de lubrifiant ;
- de 0 à 70,0 % d'ingrédients autres que du mannitol et du lubrifiant ;
les pourcentages étant exprimés en poids sec, et leur somme étant égale à 100 %.

13. Utilisation de granules tels que définis dans l'une quelconque des revendications 1 à 7 en tant qu'excipient de compression directe.

## Patentansprüche

1. Mikrokristalline nicht kugelförmige Mannitol-Granulate, **dadurch gekennzeichnet:**
- **dass** das Mannitol sowohl in α-Kristallform als auch in β-Kristallform vorliegt, wobei das α/β-Verhältnis zwischen 10/90 und 90/10 liegt; und,
- **dass** sie im Wesentlichen frei von körniger Stärke sind; und,
- **dass** sie einen durchschnittlichen Volumendurchmesser D4,3 von 60 bis 250 µm aufweisen; und
- **dass** sie eine Schüttdichte von mindestens 465 g/l haben; und,
- **dass** sie eine spezifische Oberfläche von mehr als 0,75 m²/g aufweisen.

2. Granulate nach Anspruch 1, **dadurch gekennzeichnet, dass** das α/β-Verhältnis des Mannitols 15/85 bis 85/15 beträgt.

3. Granulate nach einem der Ansprüche 1 oder 2, **dadurch gekennzeichnet, dass** sie einen durchschnittlichen Volumendurchmesser D4,3 von mehr als 80 µm aufweisen.

4. Granulate nach einem der Ansprüche 1 bis 3, **dadurch gekennzeichnet, dass** sie eine Schüttdichte von mindestens 470 g/l aufweisen.

5. Granulate nach einem der Ansprüche 1 bis 4, **dadurch gekennzeichnet, dass** sie eine spezifische Oberfläche von mehr als 0,80 m²/g aufweisen.

6. Granulate nach einem der Ansprüche 1 bis 5, **dadurch gekennzeichnet, dass** sie eine Kompressibilität zwischen 50 und 500 N aufweisen.

7. Granulate nach einem der Ansprüche 1 bis 6, **dadurch gekennzeichnet, dass** sie eine Fließzahl von 3 bis 15 Sekunden aufweisen.

8. Verfahren zur Herstellung von Mannitolgranulaten, wie in einem der Ansprüche 1 bis 7 definiert, bestehend aus:
- einem Schritt (a) einer Herstellung einer Mannitollösung;
- einem Schritt (b) eines Sprühtrocknens der in Schritt (a) erhaltenen Mannitollösung in einem mehrfachwirkenden Sprühturm (1), umfassend das Recycling von Feinteilen, **dadurch gekennzeichnet:**
- **dass** die relative Feuchtigkeit am Ausgang des Turms in einem Bereich von 5 bis 60% gewählt wird; und,
- **dass** der mehrfachwirkende Sprühturm (1) eine Kammer (2) umfasst, die aus einem zylindrischen unteren Teil (2a), umfassend ein statisches Wirbelbett (3), einem kegelstumpfförmigen Zwischenteil (2b) und einem zylindrischen oberen Teil (2c) gebildet ist; wobei der Winkel (α_{Cy}), der durch die Seitenwand des oberen zylindrischen Teils (2c) und des Dachs der Kammer (2) gebildet wird, 90° beträgt;
- einem Schritt (c) einer Gewinnung der so erhaltenen Granulate.

9. Pulverförmige Zusammensetzung, umfassend Granulate wie in einem der Ansprüche 1 bis 7 definiert.

10. Verfahren zur Herstellung von Tabletten, bestehend aus der Direktverpressung einer Pulverzusammensetzung nach Anspruch 9.

11. Tablette, gebildet aus der Pulverzusammensetzung wie in Anspruch 9 definiert oder erhältlich durch oder erhalten nach dem Verfahren wie in Anspruch 10 definiert.

12. Tablette, die im Wesentlichen frei von körniger Stärke ist, gebildet aus:
- 30,0 bis 100,0% Mannitol;
- weniger als 0,8% Schmiermittel;
- 0 bis 70,0% anderer Inhaltsstoffe als Mannitol und Schmiermittel;
wobei die Prozentsätze in Trockengewicht ausgedrückt sind und ihre Summe 100% beträgt.

13. Verwendung von Granulaten, wie in einem der Ansprüche 1 bis 7 definiert, als Hilfsstoff für die Direktverpressung.

## Claims

1. Non-spherical granules of microcrystalline mannitol, **characterized:**
- **in that** said mannitol is both in α crystalline form and in β crystalline form, the α/β ratio being between 10/90 and 90/10; and
- **in that** they are essentially free of granular starch; and
- **in that** they have a volume mean diameter D4,3 of from 60 to 250 µm; and
- **in that** they have a bulk density of at least 465 g/l; and
- **in that** they have a specific surface area greater than 0.75 m²/g.

2. The granules as claimed in claim 1, **characterized in that** the α/β ratio of said mannitol is from 15/85 to 85/15.

3. The granules as claimed in either of claims 1 and 2, **characterized in that** they have a volume mean diameter D4,3 greater than 80 µm.

4. The granules as claimed in any one of claims 1 to 3, **characterized in that** they have a bulk density of at least 470 g/l.

5. The granules as claimed in any one of claims 1 to 4, **characterized in that** they have a specific surface area greater than 0.80 m²/g.

6. The granules as claimed in any one of claims 1 to 5, **characterized in that** they have a tabletability of between 50 and 500 N.

7. The granules as claimed in any one of claims 1 to 6, **characterized in that** they have a flow grade of from 3 to 15 seconds.

8. A process for preparing mannitol granules as defined in any one of claims 1 to 7, consisting of:
- a step (a) of preparing a mannitol solution;
- a step (b) of spray-drying the mannitol solution obtained in step (a) in a multi-stage spray-drying tower (1), comprising the recycling of the fines, characterized:
o in that the relative humidity at the tower outlet is chosen in a range of from 5% to 60%; and
o in that the multi-stage spray-drying tower (1) comprises a chamber (2) consisting of a cylindrical lower part (2a) comprising a static fluidized bed (3), of a frustoconical intermediate part (2b), and of a cylindrical upper part (2c); the angle (α_{Cy}) formed by the side wall of the cylindrical upper part (2c) and the roof of the chamber (2) being equal to 90°;
- a step (c) of recovering the granules thus obtained.

9. A pulverulent composition comprising granules as defined in any one of claims 1 to 7.

10. A process for preparing tablets, consisting of the direct compression of a pulverulent composition as claimed in claim 9.

11. A tablet consisting of the pulverulent composition as defined in claim 9, or capable of being obtained by, or obtained by the process as defined in claim 10.

12. A tablet essentially free of granular starch, consisting:
- of 30.0% to 100.0% of mannitol;
- of less than 0.8% of lubricant;
- of 0% to 70.0% of ingredients other than mannitol and lubricant;
the percentages being expressed by dry weight, and their sum being equal to 100%.

13. The use of granules as defined in any one of claims 1 to 7 as direct compression excipient.
